# EUROPÄISCHE PATENTSCHRIFT

(11) **EP 1 337 512 B1**
(45) Veröffentlichungstag und Bekanntmachung des Hinweises auf die Patenterteilung: **13.06.2007**
(21) Anmeldenummer: 01978415.6
(22) Anmeldetag: 16.10.2001
(51) Int. Cl.: C07D 211/58, A61P 11/06, A61P 27/14, A61P 29/00, A61P 25/00, C07D 401/12, C07D 413/12, A61K 31/495, A61P 37/08

(54) **(4-ACYLAMINOPIPERIDIN-1-YL)ACETAMIDE ALS NEUROKININANTAGONISTEN**
(4-ACYLAMINOPIPERIDIN-1-YL) ACETAMIDES AS NEUROKININ ANTAGONISTS
(4-ACYLAMINOPIPERIDIN-1-YLE) ACETAMIDES SERVANT D'ANTAGONISTES DE NEUROKININE

(30) Priorität: 17.10.2000 DE 10051321
(43) Veröffentlichungstag der Anmeldung: 27.08.2003
(73) Patentinhaber: Boehringer Ingelheim Pharma GmbH & Co.KG, 55216 Ingelheim am Rhein (DE)
(72) Erfinder: DOLLINGER, Horst, 88433 Schemmerhofen (DE); ESSER, Franz, 55218 Ingelheim am Rhein (DE); JUNG, Birgit, 55270 Schwabenheim (DE); SCHNORRENBERG, Gerd, 88400 Biberach (DE); SCHROMM, Kurt, 55218 Ingelheim am Rhein (DE); SPECK, Georg, 55218 Ingelheim am Rhein (DE)
(86) Internationale Anmeldenummer: PCT/EP2001/011907
(87) Internationale Veröffentlichungsnummer: WO 2002/032866

(56) Entgegenhaltungen:
- WO-A-95/14017
- WO-A-96/32386

## Beschreibung

Die Erfindung betrifft neue Verbindungen der Formel I, 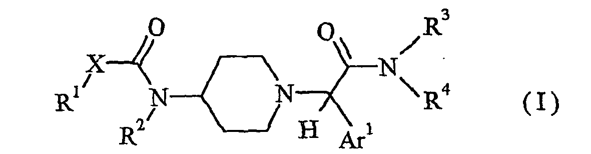 worin die Reste Ar¹, R¹, R², R³, R⁴ und X die in den Ansprüchen und in der Beschreibung genannten Bedeutungen haben, Verfahren zu deren Herstellung sowie deren Verwendung als Arzneimittel, und deren pharmazeutisch annehmbare Salze, Verfahren zu ihrer Herstellung und diese Verbindungen enthaltende pharmazeutische Zusammensetzungen. Die Verbindungen sind wertvolle Neurokinin (Tachykinin)-Antagonisten.

### Hintergrund der Erfindung

Die Verbindungen der Formel I werden teilweise von der breiten allgemeinen Formel der internationalen Patentanmeldung WO96/32386 umfasst. Jedoch werden dort keine Verbindungen offenbart, bei denen die Amidgruppe mit einem 2-Phenyl-ethylrest und der Piperidylrest in 4-Stellung mit einer substituierten Urethan- oder Harnstoffgruppe substituiert ist. Die in dieser internationalen Patentanmeldung beschriebenen Verbindungen sind Neurokinin Antagonisten mit breitem Wirkungsspektrum.

Aufgabe der vorliegenden Erfindung ist es, neue Neurokinin Antagonisten mit erhöhter Wirkung aufzuzeigen. Diese Aufgabe wurde nun erfindungsgemäß gelöst durch die Bereitstellung der neuen Verbindungen der Formel I.

### Detaillierte Beschreibung der Erfindung

Überraschenderweise wurde festgestellt, dass die Wirkung der neuen NK₁ Rezeptor Antagonisten der Formel drastisch erhöht ist gegenüber den bekannten Verbindungen.

Die Erfindung betrifft daher neue Verbindungen der Formel I 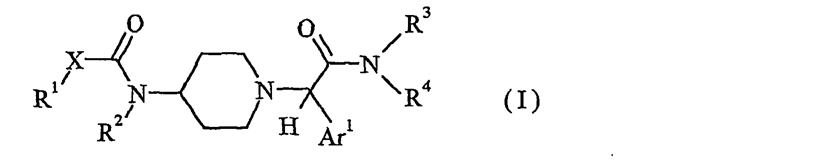 oder deren pharmazeutisch annehmbare Salze,
worin
- R¹: C₁-C₆-Alkyl oder Ar² bedeutet,
- R²: Wasserstoff, C₁-C₆-Alkyl oder C₃-C₆-Cycloalkylmethyl bedeutet, oder
- R¹ und R²: zusammengenommen eine eine gegebenenfalls durch ein oder zwei Oxogruppen (=O) substituierte C₂-C₃-Alkylendiylgruppe bedeuten,
- X: O oder NR⁵ bedeutet,
- Ar¹ und Ar²: jeweils unabhängig voneinander für unsubstituiertes Phenyl oder 1- bis 5-fach durch Halogen, Hydroxy, C₁-C₄-Alkyl, C₁-C₄-Alkoxy, C₁-C₄-Fluoroalkyl, C₁-C₄-Fluoroalkoxy oder -OCH₂O- substituiertes Phenyl steht;
- R³: für 2-Phenyl-ethyl steht, worin die Phenylgruppe durch 1 bis 3 Substituenten substituiert sein kann, worin die Substituenten unabhängig voneinander ausgewählt sind aus der Gruppe bestehend aus Halogen, Hydroxy, C₁-C₄-Alkyl, C₁-C₄-Alkoxy, C₁-C₄-Fluoroalkyl, C₁-C₄-Fluoroalkoxy;
- R⁴: für Wasserstoff, C₁-C₄-Alkyl, C₃-C₈-Cycloalkyl, CH₂COOH, -CH₂C(O)NH₂, -OH oder Phenyl-C₁-C₄-alkyl steht ; und
- R⁵: für Wasserstoff oder C₁-C₆-Alkyl steht.

Vor- und nachstehend bedeuten die Begriffe "Alkyl" und "Alkoxy wie sie bezüglich der Reste R¹, R², R³, R⁴ bzw. der Substituenten von Ar¹ oder Ar² verwendet werden, geradkettige oder verzweigte, gesättigte Kohlenwasserstoff-Reste mit bis zu 6 Kohlenstoffatomen, vorzugsweise 1 4, Kohlenstoffatomen, insbesondere Methyl, Ethyl, *n*-Propyl, *i*-Propyl, *n*-Butyl, *tert*-Butyl, Methoxy, Ethoxy, *n*-Propoxy oder *i*-Propoxy.

Vor- und nachstehend bedeuten die Begriffe "Fluoroalkyl" und "Fluoroalkoxy wie sie bezüglich de Restes R³ bzw. der Substituenten von Ar verwendet werden, geradkettige oder verzweigte, mit Fluor substituierte Kohlenwasserstoff Reste mit bis zu 4 Kohlenstoffatomen und bis zu 9 Fluoratomen, vorzugsweise 1 oder 2, Kohlenstoffatomen und bis zu 5 Fluoratomen, insbesondere Trifluorethyl, Pentafluorethyl, 2,2,2-Trifluorethyl, 2-Fluorethyl, Difluormethoxy, Trifluormethoxy Pentafluorethoxy, 2,2,2-Trifluorethoxy oder 2-Fluorethoxy.

Die erfindungsgemäßen Verbindungen sind wertvolle Neurokinin (Tachykinin)- Antagonisten, die Substanz P-antagonistische Eigenschaften besitzen. Sie sind nützlich zur Behandlung von und zur Vorbeugung gegenüber Neurokinin-vermittelten Krankheiten und besitzen zudem eine drastisch erhöhte Wirkung.

Verbindungen der allgemeinen Formel I können Säuregruppen besitzen, hauptsächlich Carboxylgruppen, und/oder basische Gruppen wie z.B. Aminofunktionen. Verbindungen der allgemeinen Formel I können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwend-baren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die erfindungsgemäßen Verbindungen können als Racemate vorliegen, sie können aber auch als reine Enantiomere, d.h. in (R)- oder (S)-Form gewonnen werden. Bevorzugt sind Verbindungen die als Racemate bzw. als (S)-Form vorliegen.

Die erfindungsgemäßen Verbindungen sind wertvolle Neurokinin (Tachykinin)-Antagonisten, die Substanz P-antagonistische Eigenschaften besitzen. Sie sind nützlich zur Behandlung von und zur Vorbeugung gegenüber Neurokinin-vermittelten Krankheiten:
Behandlung oder Vorbeugung von entzündlichen und allergischen
Erkrankungen
der Atemwege, wie Asthma, chronische Bronchitis, hyperreagible Atemwege, Emphysem, Rhinitis, COPD, pulmonale Hypertonie, cystische Fibrose, Husten;
der Augen, wie Konjunktivitis und Iritis,
der Haut, wie Dermatitis bei Kontaktekzem, Neurodermitis, Pruritus, Urtikaria, Psoriasis,
Sonnenbrand, Verbrennungen, Insektenstiche, Rosazea, Juckreiz, sensible oder überempfindliche Haut,
des Magen-Darm-Traktes, wie Magen- und Duodenalgeschwüre, Colitis Ulcerosa, Morbus Crohn, entzündliche Darmerkrankung, Colon irritabile, M. Hirschsprung, Mobil itätsstörungen;
der Gelenke oder Knochen, wie rheumatoide Arthritis, reaktive Arthritis, Arthrose, Osteoporose und Reiter-Syndrom;
der Blase, wie Reizblase, Inkontinenz, Harndrang, Urethritis, Kolik und Zystitis.

Weiterhin zur Behandlung von Erkrankungen des Zentralnervensystems, wie Demenz, M. Alzheimer, Schizophrenie, Psychosen, Angstzustände, Alkohol- oder Drogenabhängigkeit, Sexuelle Dysfunktionen, Essstörungen, Depression, Kopfschmerzen (z.B. Migräne oder Spannungskopfschmerzen), Epilepsie; M. Parkinson, Schlaganfall, Behandlung von Herpes zoster sowie postherpetischer Schmerzen, von Tumoren, Kollagenosen, einer Dysfunktion der ableitenden Harnwege, von Hämorrhoiden, von Übelkeit und Erbrechen, ausgelöst z.B. durch Bestrahlung oder Zytostatikatherapie oder Bewegung und Schmerzzuständen aller Art.

Die Erfindung betrifft daher auch die Verwendung der Verbindungen der Formel I als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch Iontophorese oder literaturbekannte Enhancer gefördert, und oral erfolgen.

Zur parenteralen Applikation werden die Verbindungen der Formel I oder deren physiologisch veträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Bevorzugt sind Verbindungen der Formel I, worin R⁴ C₁-C₄-Alkyl, insbesondere Methyl ist.

Weiterhin bevorzugt sind Verbindungen der Formel I, worin Ar unsubstituiertes Phenyl oder 2,3-Methylendioxyphenyl, insbesondere unsubstituiertes Phenyl ist.

Solche Verbindungen der Formel I sind bevorzugt, wobei R³ für 2-Phenylethyl steht, worin die Phenylgruppe durch 1 bis 3 Substituenten substituiert sind, wobei die Substituenten jeweils unabhängig voneinander ausgewählt sind aus der Gruppe bestehend aus Halogen, Hydroxy, Methyl Methoxy, Trifluormethyl, Trifluormethoxy, insbesondere worin R³ 2-(3,5-Bis-trifluormethylphenyl)-ethyl ist.

Verbindungen der Formel I sind besonders bevorzugt, worin die Gruppe -NR³R⁴ 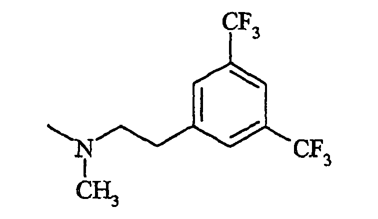 ist.

Ein bevorzugter Aspekt der Erfindung betrifft Verbindungen der Formel I, worin
R¹ eine C₁-C₃-Alkyl, insbesondere Methyl, Phenyl oder C₁-C₃-Alkoxyphenylgruppe, insbesond 4-Methoxyphenyl, bedeutet,
X für NH, und
R² für ein Wasserstoffatom steht.

Ein weiterer bevorzugter Aspekt der Erfindung betrifft Verbindungen der Formel I, worin R¹ und R² zusammengenommen eine Ethylen-1,2-diyl, 1-Oxoethylen-1,2-diyl, Propylen-1,3-diyl, 1 Oxopropylen-1,3-diyl oder 1-Oxobutylen-1,3-diyl gruppe bedeuten, und X für O, NH oder NCH₃ steht.

Besonders bevorzugt sind NK1 Rezeptor Antagonisten der Formel I, worin der Rest

R¹-X-CO-NR²-

eine Gruppe ausgewählt aus den Formeln A-1 bis A-8 ist:

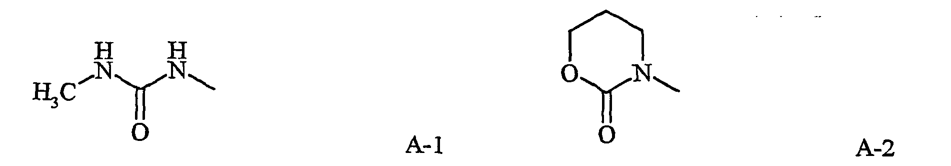

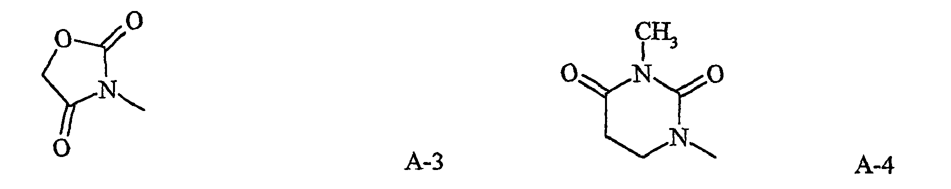

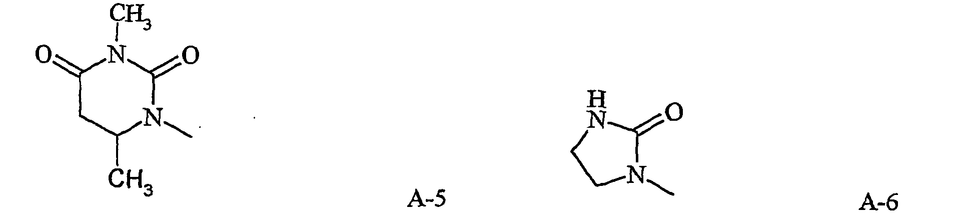

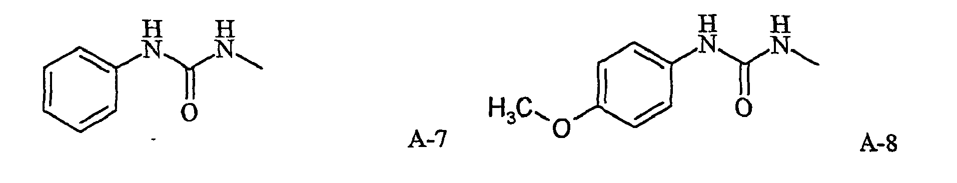

Besonders bevorzugt sind folgende Verbindungen:

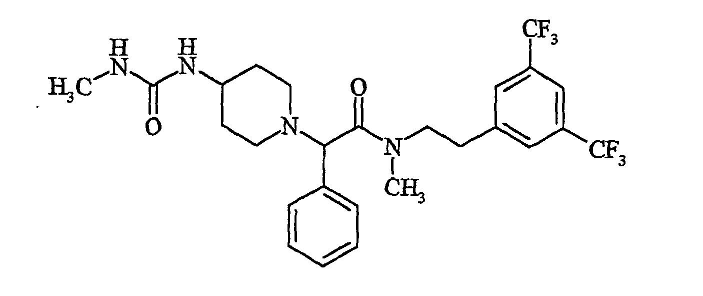

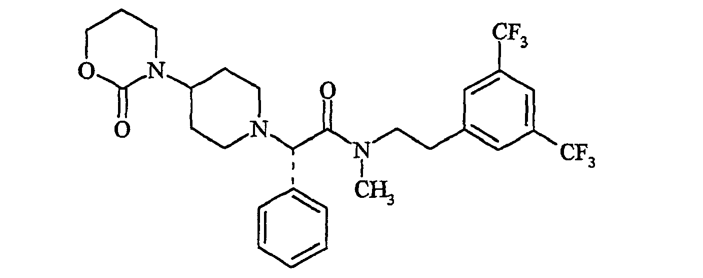

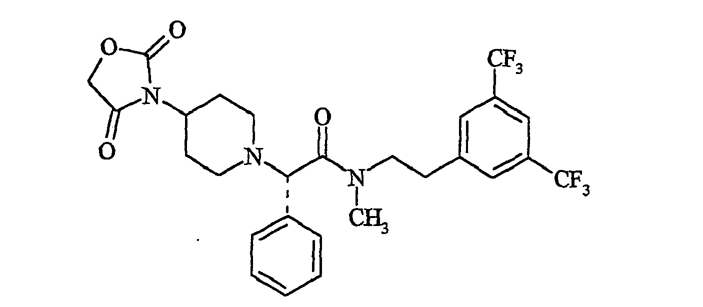

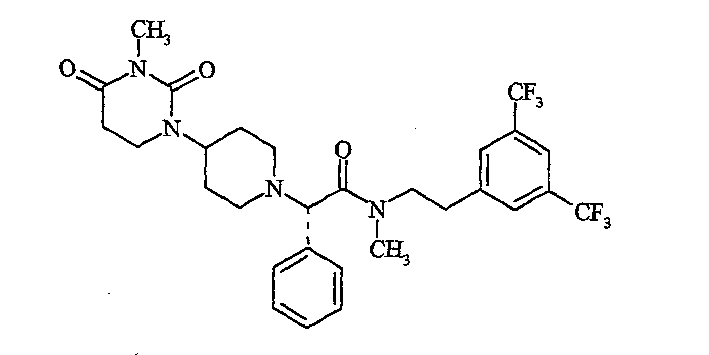

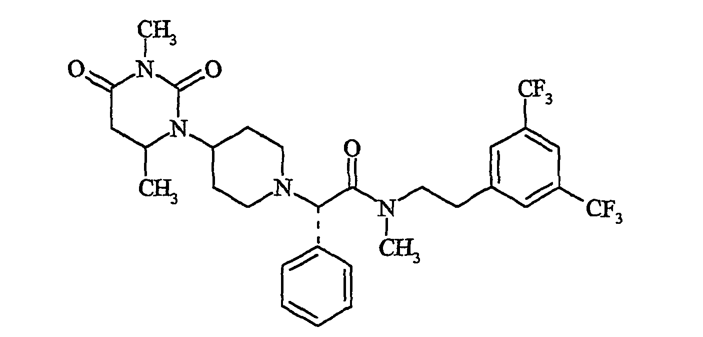

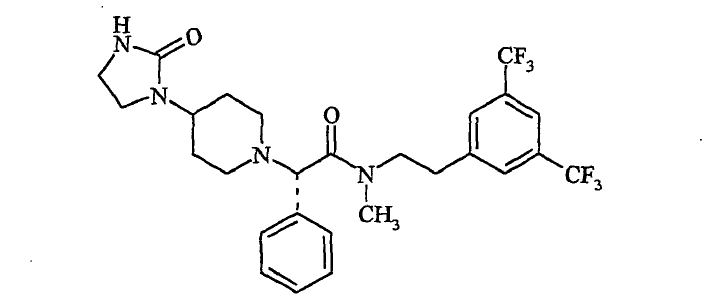

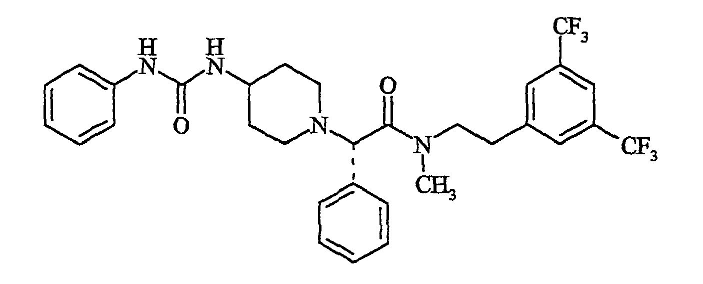

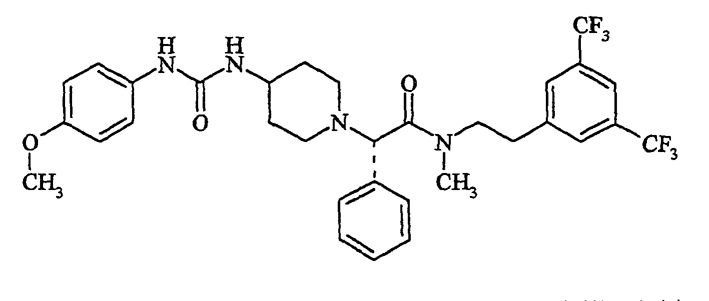

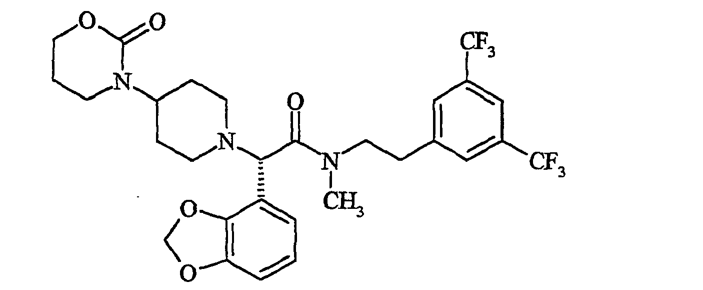

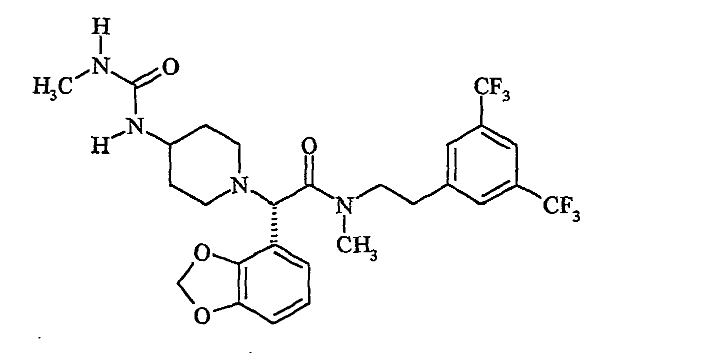

Die Herstellung der Verbindungen kann auf an sich bekannte Weise erfolgen. Vorteilhafte Methoden sind in dem folgenden Schema dargestellt und beschrieben. Die Verbindungen der allgemeinen Formel I können hergestellt werden, indem man ein Amid der Formel II 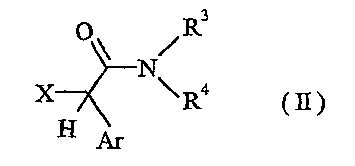 worin X eine geeignete Abgangsgruppe, vorzugsweise Halogen, Alkylsulfonyloxy, insbesondere Methylsulfonyloxy, oder Arylsulfonyloxy, insbesondere p-Tolylsulfonyloxy, bedeutet, in einem inerten Lösungsmittel in Gegenwart einer Base mit einem Piperidin der allgemeinen Formel III 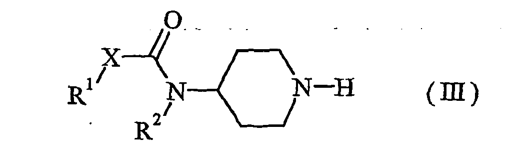 umsetzt.

Dieses Verfahren wird anhand des folgenden Schemas 1 für Verbindungen dargestellt, in denen Ar Phenyl, R³ Bis-(trifluoromethyl)-phenylethyl und R⁴ Methyl ist. Das Verfahren ist jedoch für alle Verbindungen der Formel I analog anwendbar. Die Verbindungen der Formel III sind bekannt oder können in Analogie zu an sich bekannten Verfahren hergestellt werden.

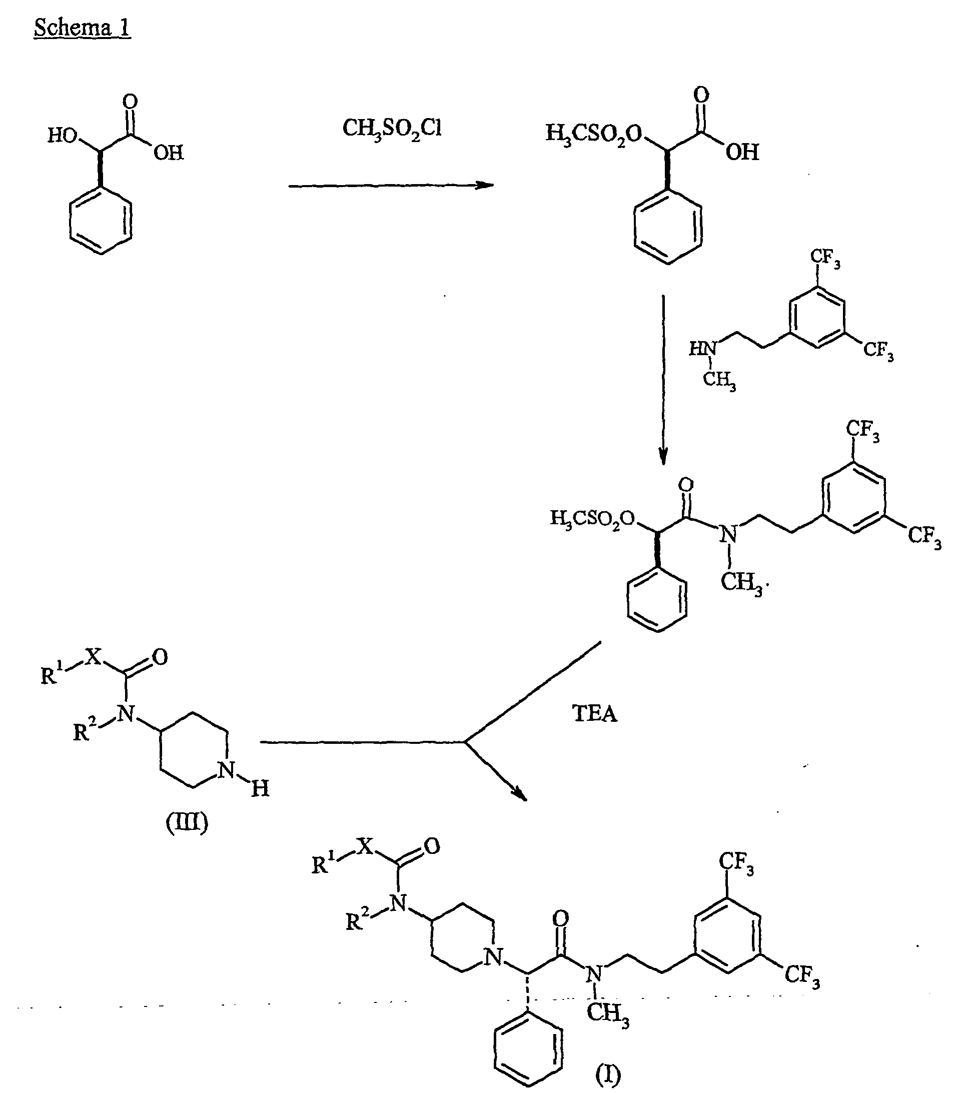

Den Reaktionspartner für dieses Piperazinderivat erhält man wie im Schema 1, rechts dargestellt. (R)-Mandelsäure wird dabei mit Methansulfonsäurehalogenid umgesetzt zur (R)-2-(Methansulfonyloxy)-essigsäure. Diese wird nun mit einem Kupplungsreagens und dem entsprechend substituierten Phenethylamin umgesetzt zum entsprechenden Amid, oder sie wird in das entsprechende Säurehalogenid überführt (z.B. mit SOCl₂/SO₂Cl₂) und dann mit dem geeignet substituierten Phenethylamin in das entsprechende Amid umgewandelt. Im letzten Schritt wird das so erhaltene Amid umgesetzt mit dem oben beschriebenen Piperidinderivat wobei es unter Substitution von Methansulfonat zur C-N-Knüpfung kommt unter gleichzeitiger Umkehr des Chiralitätszentrums. Die Umsetzung erfolgt in einem inerten Lösungsmittel, vorzugsweise einem polar aprotischen Lösungsmittel wie z.B. DMF, Dimethylacetamid, Ethylmethylketon oder Acetonitril in Gegenwart einer Base, vorzugsweise einer anorganischen Base wie z.B. K₂CO₃, NaHCO₃ oder CaCO₃, oder organische Basen wie z.B. tertiäre Amine, vorzugsweise Triethylamin, Hünig-Base, Pyridin oder N-Methylmorpholin, zwischen 0 °C und 120 °C, typischerweise zwischen 10 °C und 80°C. Die Reaktionszeit liegt in der Regel zwischen 0,5 h und 48 h.

Die erfindungsgemäßen Verbindungen und Zusammensetzungen sollen nun durch die nachfolgenden Beispiele erläutert werden. Dem Fachmann ist bewusst, dass die Beispiele nur zur Veranschaulichung dienen und als nicht limitierend anzusehen sind.

### A Synthesebeispiel erfindungsgemäßer Verbindungen

### Beispiel 1

### N-[2-(3,5-Bis-trifluormethyl-phenyl)-ethyl]-N-methyl-2-[4-(3-methyl-nreiäo)-piperidin-1 -yl]-2-phenyl-acetamid

6,8 g 4-(3-Methylureido)-piperidin werden zusammen mit 19,2 g N-[2-(3,5-Bis-trifluormethyl-phenyl)-ethyl]-2-methansulfonyloxy-N methyl-2-phenyl-acetamid (hergestellt analog wie in WO 99/62893 beschrieben) und 6,8 ml Triethylamin in 400 ml Aceton 8 Stunden unter Rückfluss gekocht. Anschließend wird die Lösung eingeengt, mit ges. Natriumhydrogen-carbonat-Lösung versetzt und mit Ethylacetat extrahiert. Der Extrakt wird getrocknet, im Vakuum vom Lösungsmittel befreit und der Rückstand mit Methylenchlorid / Methanol 9:1 über Kieselgel chromatographiert. Die im DC einheitlichen Fraktionen werden vereinigt und im Vakuum vom Lösungsmittel befreit. Aus dem Rückstand wird mit ethanolischer Salzsäure und Ether N-[2-(3,5-Bis-tifluormethyl-phenyl)-ethyl]-N-methyl-2-[4-(3-methyl-ureido)-piperidin-1-yl]-2-phenyl-acetamid als Hydrochlorid kristallisiert, man erhält 7,1 g farblose Kristalle.
¹H-NMR (250 MHz, CD₃OD) δ ppm = 7,93-7,33 (8H, m); 5,58; 5,38 (1H, 2s); 4,03- 2,59 (5H, m); 3,05 (4H, m); 2,98; 2,90 (3H, 2s); 2,74; 2,70 (3H, 2s); NH im Lösungsmittel-Blindpeak 4,89; 2,27-1,52 (4H, m). Die meisten Signale aufgrund Amidrotation aufgespalten.

### Beispiel 2

### (S)-N-[2-(3,5-Bis-trifluormethyl-phenyl)-ethyl]-N-methyl-2-[4-(2-oxo-[1,3]oxazinan-3-yl)-piperidin-1-yl]-2-phenyl-acetamid

5,4 g 4-(2-Oxo-[1,3]oxazinan-3-yl)-piperidin werden zusammen mit 12,5 g (R)-N-[2-(3,5-Bis-trifluormethyl-phenyl)-ethyl]-2-methansulfonyloxy-N-methyl-2-phenyl-acetamid (hergestellt aus D-(-)-Mandelsäure) und 4,5 ml Triethylamin in 250 ml Aceton 6 Stunden unter Rückfluss gekocht. Anschließend wird die Lösung eingeengt, mit ges. Natriumhydrogencarbonat-Lösung versetzt und mit Ethylacetat extrahiert. Der Extrakt wird getrocknet, im Vakuum vom Lösungsmittel befreit und der Rückstand mit Methylenchlorid / Methanol 9:1 über Kieselgel chromatographiert. Die im DC einheitlichen Fraktionen werden vereinigt und im Vakuum vom Lösungsmittel befreit. Aus dem Rückstand wird mit ethanolischer Salzsäure und Ether (S)-N-[2-(3,5-Bis-trifluormethyl-phenyl)-ethyl]-N-methyl-2-[4-(2-oxo-[1,3]oxazinan-3-yl)-piperidin-1-yl]-2-phenyl-acetamid als Hydrochlorid kristallisiert. Man erhält 7,5 g hellbeige Kristalle.
¹H-NMR (250 MHz, CD₃OD) δ ppm = 7,88 - 7,55 (8H, m); 5,47; 5,28 (1H, 2s); 4,24 (2H, t, J = 5,8 Hz); 4,13 (1H, m); 4,06 - 2,69 (6H, m); 3,04 (4H, m); 3,00; 2,89 (3H, 2s); 2,34 - 1,71 (6H, m). Die meisten Signale aufgrund Amidrotation aufgespalten.
Drehwert [α]_{D}²⁰ = +36,7° (c=1, Methanol)

In analoger Weise können die Beispiele 3 bis 10 hergestellt werden.

| | | | | |
|---|---|---|---|---|
| 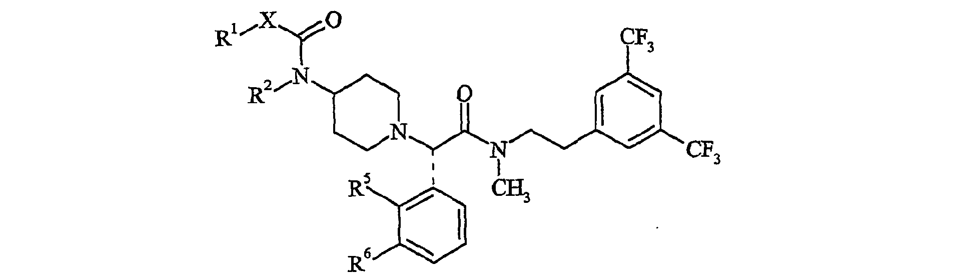 | | | | |

| Beispiel | R¹-X- | R²- | R⁵ | R⁶ |
|---|---|---|---|---|
| 3 | -O-CH₂-C=O- | | H | H |
| 4 | -N(CH₃)-C=O-CH₂CH₂- | | H | H |
| 5 | -N(CH₃)-C=O-CH₂CH(CH₃)- | | H | H |
| 6 | -NH-CH₂CH₂- | | H | H |
| 7 | Phenyl-NH- | H- | H | H |
| 8 | 4-Methoxy-phenyl-NH- | H- | H | H |
| 9 | -O-CH₂-CH₂CH₂- | | -O-CH₂-O- | |
| 10 | Methyl-NH- | H | -O-CH₂-O- | |

### B Untersuchungsergebnisse für erfindungsgemäße Verbindung:

Die Rezeptoraffinität zum NK₁-Rezeptor (Substanz P-Rezeptor) wird an humanen Lymphoblastoma-Zellen (IM-9) mit klonierten NK₁-Rezeptoren bestimmt, wobei die Verdrängung von ¹²⁵J-markierter Substanz P gemessen wird. Die so erhaltenen Kᵢ-Werte zeigen die Wirksamkeit der Verbindungen.

Dabei wurden die erfindungsgemäßen Verbindungen mit den aus der internationalen Patentanmeldung WO96/32386 bekannten Verbindungen der Formeln: 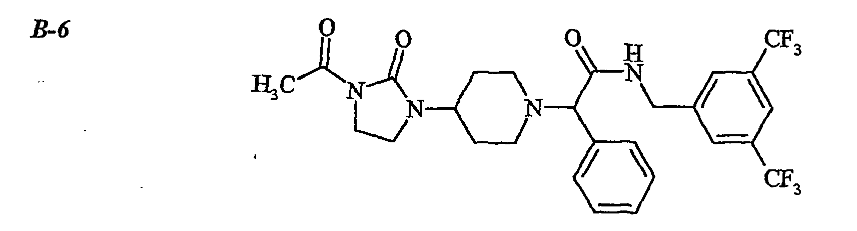 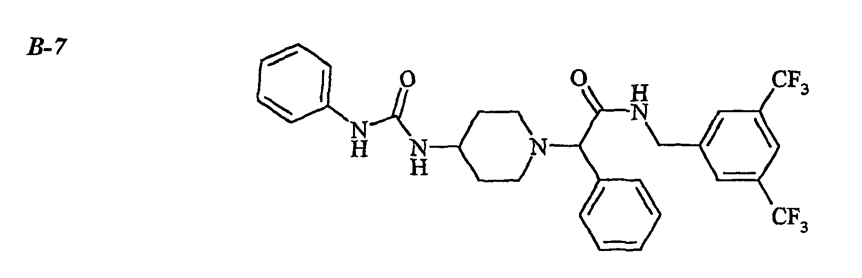 verglichen. Diese Verbindungen entsprechen den Verbindungen der Beispiele 6 und 7, worin die 2 Bis-trifluormethylphenyl-ethylgruppe durch eine Bis-trifluormethylbenzyl ersetzt wurde.

Die Ergebnisse sind in Tabelle I aufgeführt

| Beispiel Nr. | Kᵢ [nM] |
|---|---|
| 1 | 0,7 |
| 2 | 1,7 |
| 3 | 0, 7 |
| 4 | 0,6 |
| 5 | 0,6 |
| 6 | 3,5 |
| ***B-6*** | 165,0 |
| 7 | 0,8 |
| ***B-7*** | 432,0 |

### C Formulierungen erfindungsgemäßer Verbindungen

| Injektionslösung | | | |
|---|---|---|---|
| 200 mg | Wirksubstanz * | | |
| 1,2 mg | Monokaliumdihydrogenphosphat = KH₂PO₄ | ) | |
| 0,2 mg | Dinatriumhydrogenphosphat = | ) | (Puffer) |
| | NaH₂PO₄.2H₂O | | ) |
| 94 mg | Natriumchlorid | ) | (Isotonans) |
| oder | | | |
| 520 mg | Glucose | ) | |
| 4 mg | Albumin | | (Proteasenschutz) |
| q.s. | Natronlauge | | |
| q.s. | Salzsäure | ) | ad pH 6 |
| ad 10 ml | Wasser für Injektionszwecke | | |

| Injektionslösung | | | |
|---|---|---|---|
| 200 mg | Wirksubstanz* | | |
| 94 mg | Natriumchlorid | | |
| oder | | | |
| 520 mg | Glucose | | |
| 4 mg | Albumin | | |
| q.s. | Natronlauge | ) | |
| q.s. | Salzsäure | ) | ad pH 9 |
| ad 10 ml | Wasser für Injektionszwecke | | |

| Lyophilisat | |
|---|---|
| 200 mg | Wirksubstanz* |
| 520 mg | Mannit (Isotonans/Gerüstbildner) |
| 4 mg | Albumin |
| Lösungsmittel | 1 für Lyophilisat |
| 10 ml | Wasser für Injektionszwecke |
| Lösungsmittel | 2 für Lyophilisat |
| 20 mg | Polysorbat^{®}80 = Tween^{®}80 |
| | (oberflächenaktiver Stoff) |
| 10 ml | Wasser für Injektionszwecke |

| | |
|---|---|
| * Wirksubstanz: erfindungsgemäße Verbindung, z.B. eine der Beispiele 1 bis 8 Dosis für Mensch von 67 kg: 1 bis 500 mg | |

## Patentansprüche

1. Verbindung der Formel I 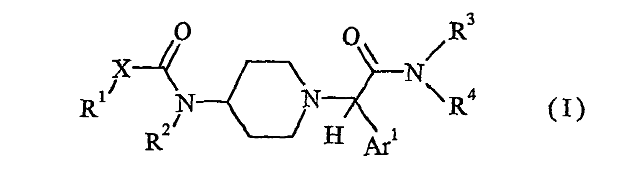 oder deren pharmazeutisch annehmbare Salze,
worin
R¹ C₁-C₆-Alkyl oder Ar² bedeutet,
R² Wasserstoff, C₁-C₆-Alkyl oder C₃-C₆-Cycloalkylmethyl bedeutet, oder
R¹ und R² zusammengenommen eine gegebenenfalls durch ein oder zwei Oxogruppen (=O) substituierte C₂-C₃-Alkylendiylgruppe bedeuten,
X O oder NR⁵ bedeutet,
Ar¹ und Ar² jeweils unabhängig voneinander für unsubstituiertes Phenyl oder 1- bis 5-fach durch Halogen, Hydroxy, C₁-C₄-Alkyl, C₁-C₄-Alkoxy, C₁-C₄-Fluoroalkyl, C₁-C₄-Fluoroalkoxy oder -OCH₂O- substituiertes Phenyl steht;
R³ für 2-Phenyl-ethyl steht, worin die Phenylgruppe durch 1 bis 3 Substituenten substituiert sein kann, worin die Substituenten unabhängig voneinander ausgewählt sind aus der Gruppe bestehend aus Halogen, Hydroxy, C₁-C₄-Alkyl, C₁-C₄-Alkoxy, C₁-C₄-Fluoroalkyl, C₁-C₄-Fluoroalkoxy;
R⁴ für Wasserstoff C₁-C₄-Alkyl, C₃-C₈-Cycloalkyl, CH₂COOH, -CH₂C(O)NH₂, -OH oder Phenyl-C₁-C₄-alkyl steht; und
R⁵ für Wasserstoff oder C₁-C₆-Alkyl steht.

2. Verbindung nach Anspruch 1, worin R⁴ C₁-C₄-Alkyl ist

3. Verbindung nach Anspruch 1 oder 2, worin Ar¹ unsubstituiertes Phenyl oder 2,3-Methylendioxyphenyl ist.

4. Verbindung nach einem der Ansprüche 1 bis 3, wobei R³ für 2-Phenylethyl steht, worin die Phenylgruppe durch 1 bis 3 Substituenten substituiert ist, wobei die Substituenten jeweils unabhängig voneinander ausgewählt sind aus der Gruppe bestehend aus Halogen, Hydroxy, Methyl Methoxy, Trifluormethyl, Trifluormethoxy.

5. Verbindung nach einem der Ansprüche 1 bis 4, worin R³ 2-(3,5-Bis-tritluormethylphenyl)-ethyl ist.

6. Verbindung nach einem der Ansprüche 1 bis 5, worin die Gruppe -NR³R⁴ 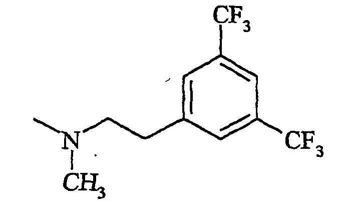 ist.

7. Verbindung nach einem der Ansprüche 1 bis 6, worin
R¹ eine C₁-C₃-Alkyl, Phenyl oder C₁-C₃-Alkoxyphenylgruppe bedeutet,
X für NH, und
R² für ein Wasserstoffatom steht.

8. Verbindung nach einem der Ansprüche 1 bis 6, worin
R¹ und R² zusammengenommen eine Ethylen-1,2-diyl, 1-Oxoethylen-1,2-diyl, Propylen-1,3-diyl, Oxopropylen-1,3-diyl oder 1-Oxobutylen-1,3-diyl gruppe bedeuten, und
X für O, NH oder NCH₃ steht.

9. Verbindung nach einem der Ansprüche 1 bis 8, ausgewählt aus den Verbindungen der Formeln

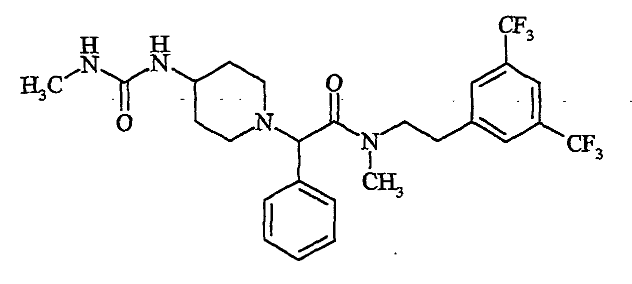

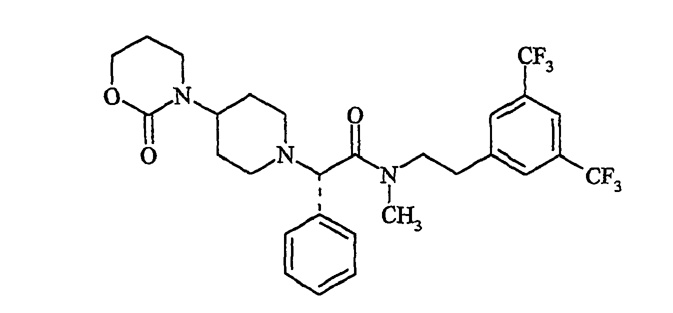

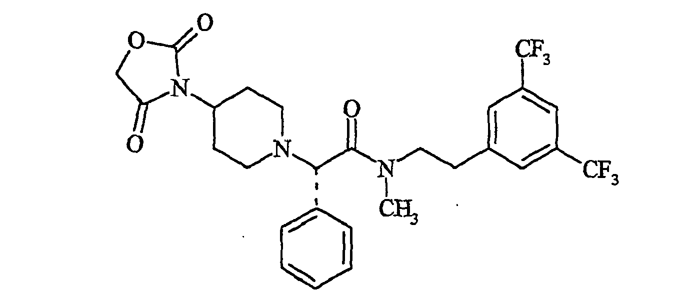

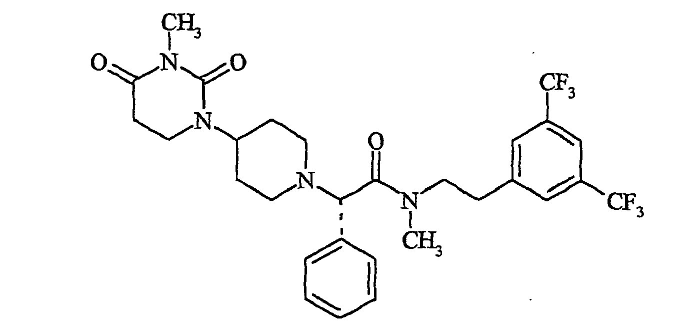

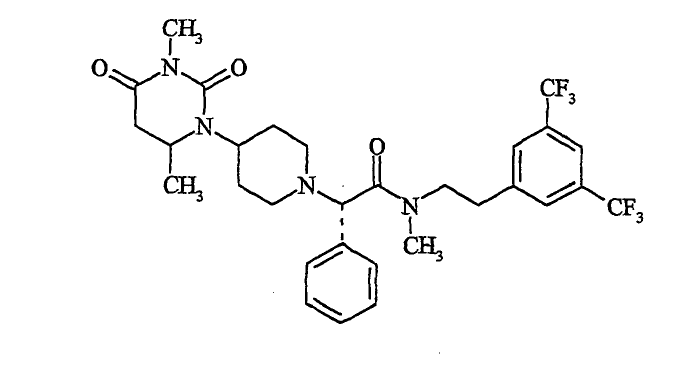

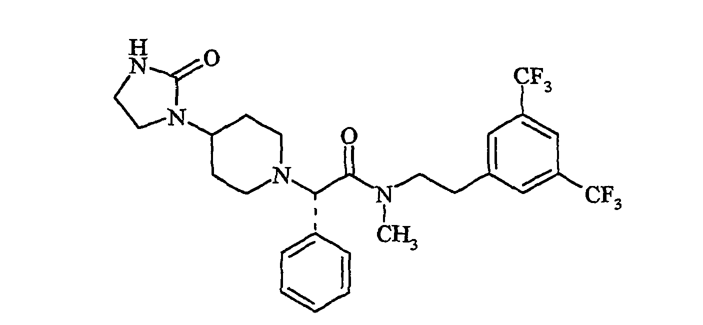

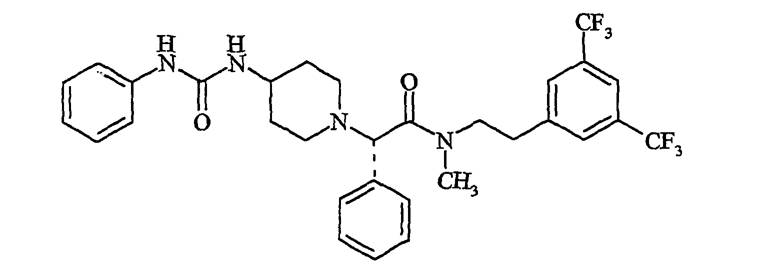

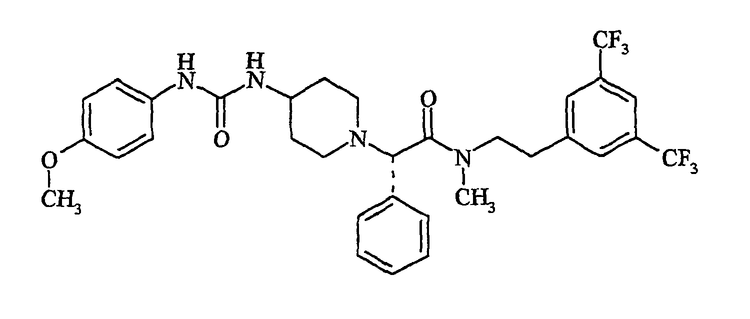

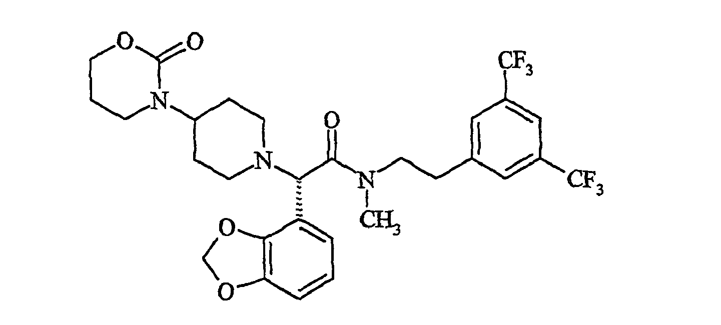

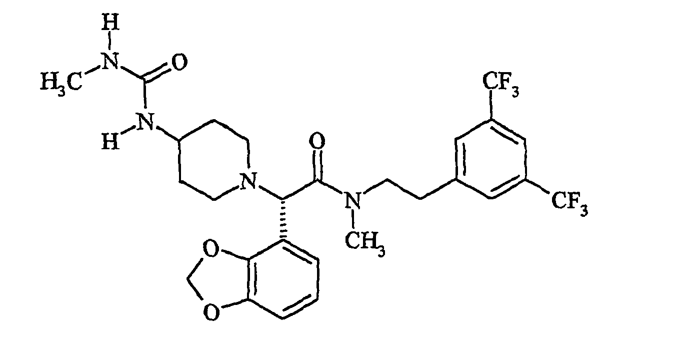

10. Verbindung nach einem der Ansprüche 1 bis 9 zur Verwendung als Arzneimittel.

11. Verfahren zur Herstellung einer Verbindung der Formel I nach einem der Ansprüche 1 bis 9, **dadurch gekennzeichnet, daß** man ein Amid der Formel II 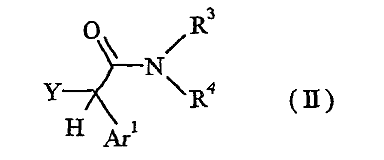 worin Ar¹, R³ und R⁴ die in den Ansprüchen 1 bis 9 angegebenen Bedeutungen aufweisen, und Y eine geeignete Abgangsgruppe bedeutet,
in einem inerten Lösungsmittel gegebenenfalls in Gegenwart einer Base mit einem Piperidin der allgemeinen Formel III 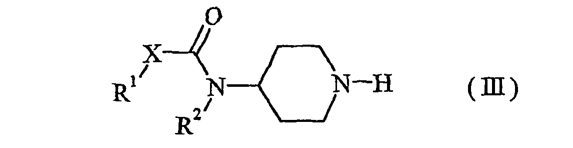 worin R¹, R² und X die in den Ansprüchen 1 bis 9 angegebenen Bedeutungen aufweisen,
umsetzt.

12. Pharmazeutische Zubereitung enthaltend eine Verbindung nach einem der Ansprüche 1 bis 9 und pharmazeutisch annehmbare Träger und Excipienten.

13. Verwendung einer Verbindung nach einem der Ansprüche 1 bis 9 für die Herstellung einer pharmazeutischen Zubereitung zur Therapie von und zur Vorbeugung gegenüber Neurokinin-vermittelten Krankheiten.

## Claims

1. Compound of formula I 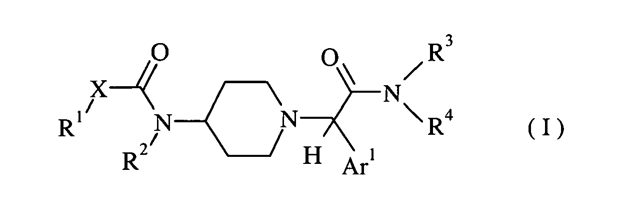 or the pharmaceutically acceptable salts thereof,
wherein
R¹ denotes C₁-C₆-alkyl or Ar²,
R² denotes hydrogen, C₁-C₆-alkyl or C₃-C₆-cycloalkylmethyl, or
R¹ and R² taken together denote a C₂-C₃-alkylenediyl group optionally substituted by one or two oxo groups (=O),
X denotes O or NR⁵,
Ar¹ and Ar² independently of one another denote unsubstituted phenyl or phenyl which is 1- to 5-substituted by halogen, hydroxy, C₁-C₄-alkyl, C₁-C₄-alkoxy, C₁-C₄-fluoroalkyl, C₁-C₄-fluoroalkoxy or -OCH₂O-;
R³ denotes 2-phenyl-ethyl, wherein the phenyl group may be substituted by 1 to 3 substituents, wherein the substituents, independently of one another, are selected from among halogen, hydroxy, C₁-C₄-alkyl, C₁-C₄-alkoxy, C₁-C₄-fluoroalkyl, C₁-C₄-fluoroalkoxy;
R⁴ denotes hydrogen, C₁-C₄-alkyl, C₃-C₈-cycloalkyl, CH₂COOH, -CH₂C(O)NH₂, -OH or phenyl-C₁-C₄-alkyl ; and
R⁵ denotes hydrogen or C₁-C₆-alkyl.

2. Compound according to claim 1, wherein R⁴ is C₁-C₄-alkyl.

3. Compound according to claim 1 or 2, wherein Ar¹ is unsubstituted phenyl or 2,3-methylenedioxyphenyl.

4. Compound according to one of claims 1 to 3, wherein R³ denotes 2-phenylethyl, in which the phenyl group is substituted by 1 to 3 substituents, wherein the substituents independently of one another are selected from among halogen, hydroxy, methyl, methoxy, trifluoromethyl and trifluoromethoxy.

5. Compound according to one of claims 1 to 4, wherein R³ is 2-(3,5-bistrifluoromethylphenyl)-ethyl.

6. Compound according to one of claims 1 to 5, wherein the group -NR³R⁴ is

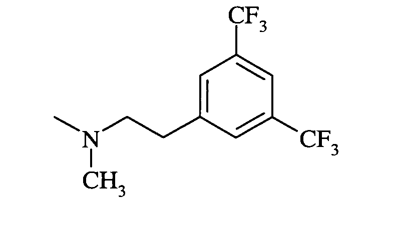

7. Compound according to one of claims 1 to 6, wherein
R¹ denotes a C₁-C₃-alkyl, phenyl or C₁-C₃-alkoxyphenyl group,
X denotes NH, and
R² denotes a hydrogen atom.

8. Compound according to one of claims 1 to 6, wherein
R¹ and R² taken together denote an ethylene-1,2-diyl, 1-oxoethylene-1,2-diyl, propylene-1,3-diyl, oxopropylene-1,3-diyl or 1-oxobutylene-1,3-diyl group, and
X denotes O, NH or NCH₃.

9. Compound according to one of claims 1 to 8, selected from among the compounds of formulae

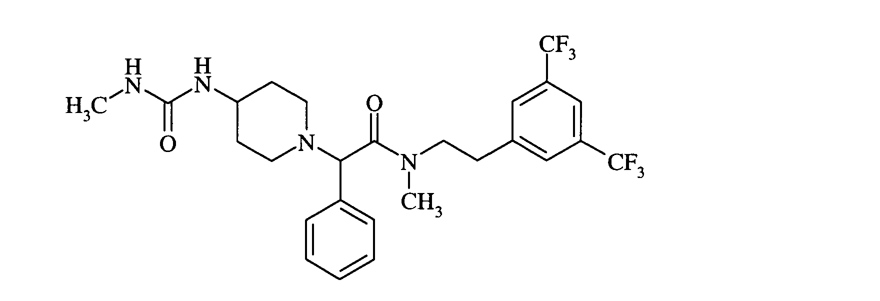

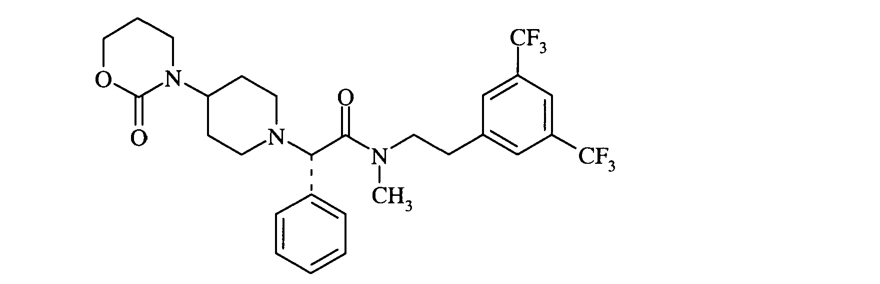

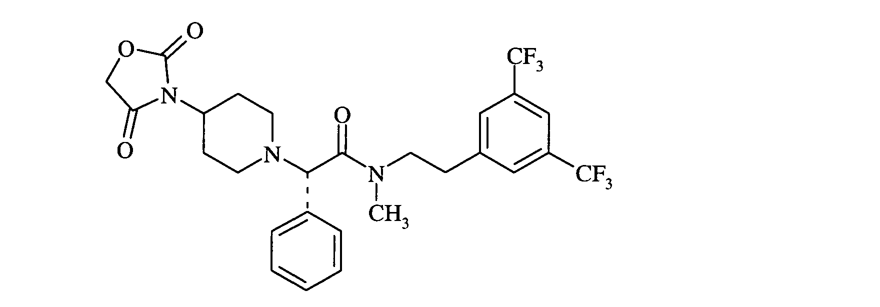

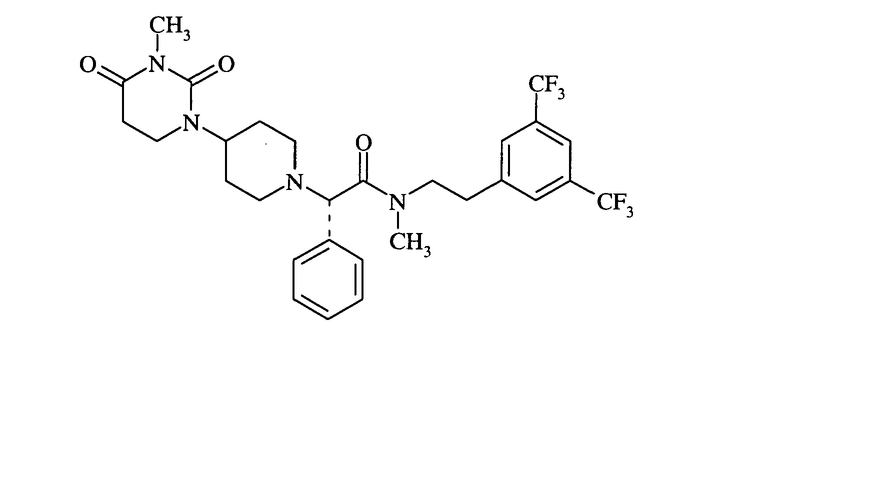

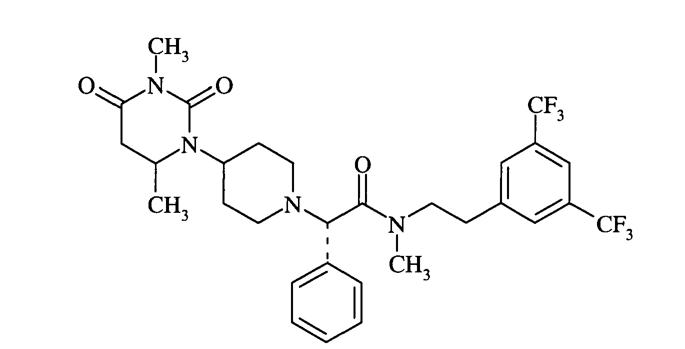

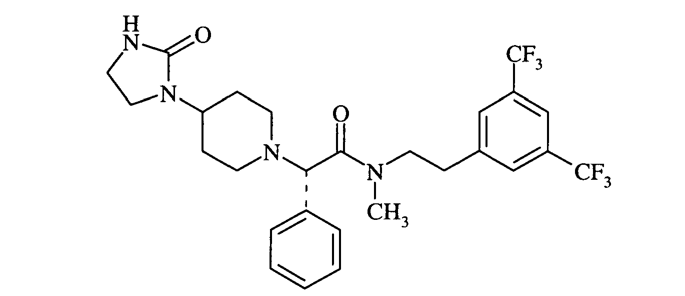

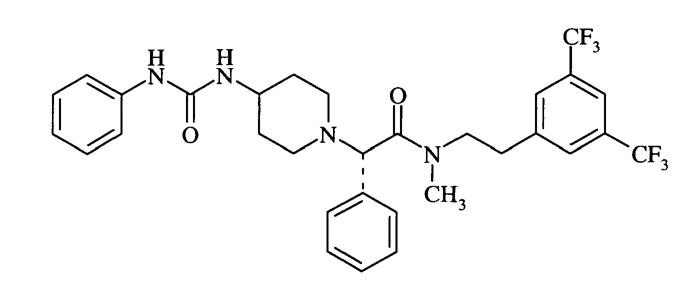

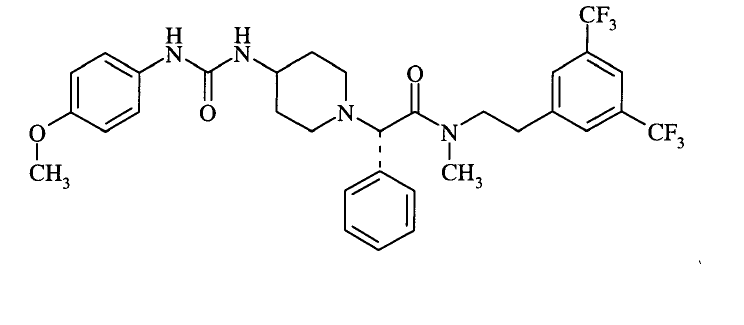

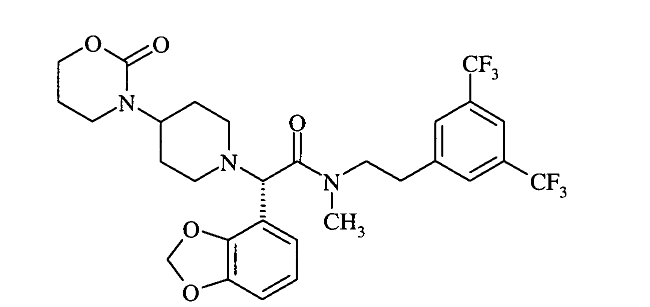

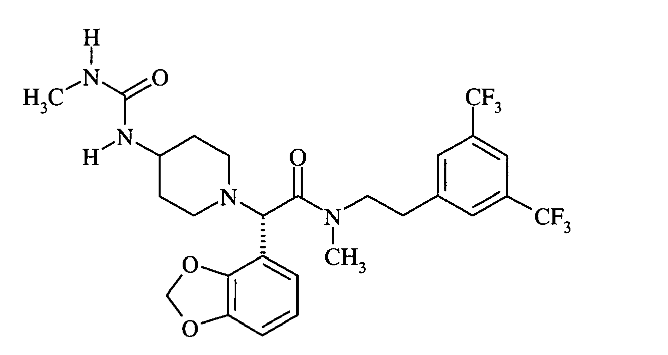

10. Compound according to one of claims 1 to 9 for use as a medicament.

11. Process for preparing a compound of formula I according to one of claims 1 to 9, **characterised in that** an amide of formula II 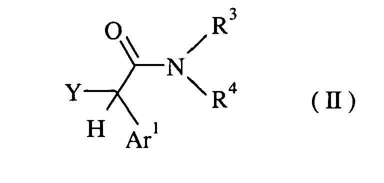
wherein Ar¹, R³ and R⁴ have the meanings given in claims 1 to 9, and
Y denotes a suitable leaving group,
is reacted with a piperidine of general formula III 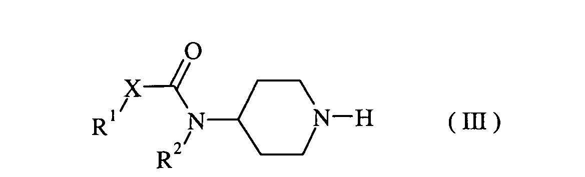 wherein R¹, R² and X have the meanings given in claims 1 to 9,
in an inert solvent, optionally in the presence of a base.

12. Pharmaceutical preparation containing a compound according to one of claims 1 to 9 and pharmaceutically acceptable carriers and excipients.

13. Use of a compound according to one of claims 1 to 9 for preparing a pharmaceutical preparation for the treatment and prevention of neurokinin-mediated illnesses.

## Revendications

1. Composé de formule **I** 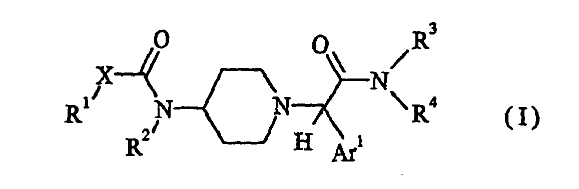 ou ses sels pharmaceutiquement acceptables
où
R¹ représente C₁-C₆-alkyle ou Ar²,
R² représente l'hydrogène, C₁-C₆-alkyle ou C₃-C₆-cycloalkylméthyle, ou
R¹ et R² combinés représentent un groupe C₂-C₃-alkylènediyle éventuellement substitué par un ou deux groupes oxo (=O),
X représente O ou NR⁵,
Ar¹ et Ar² représentent chacun indépendamment l'un de l'autre phényle non substitué ou phényle substitué 1 à 5 fois par halogène, hydroxy, C₁-C₄-alkyle, C₁-C₄-alcoxy, C₁-C₄-fluoroalkyle, C₁-C₄-fluoroalcoxy ou -OCH₂O- ;
R³ représente 2-phényl-éthyle, où le groupe phényle peut être substitué par 1 à 3 substituants, où les substituants sont choisis indépendamment les uns des autres dans le groupe consistant en halogène, hydroxy, C₁-C₄-alkyle, C₁-C₄-alcoxy, C₁-C₄-fluoroalkyle, C₁-C₄-fluoroalcoxy ;
R⁴ représente l'hydrogène, C₁-C₄-alkyle, C₃-C₈-cycloalkyle, CH₂COOH, -CH₂C(O)NH₂, -OH ou phényl-C₁-C₄-alkyle ; et
R⁵ représente l'hydrogène ou C₁-C₆-alkyle.

2. Composé selon la revendication 1 où R⁴ est C₁-C₄-alkyle.

3. Composé selon la revendication 1 ou 2 où Ar¹ est phényle non substitué ou 2,3-méthylènedioxyphényle.

4. Composé selon l'une des revendications 1 à 3 où R³ représente 2-phényléthyle, où le groupe phényle est substitué par 1 à 3 substituants, où les substituants sont choisis chacun indépendamment les uns des autres dans le groupe consistant en halogène, hydroxy, méthyle, méthoxy, trifluorométhyle, trifluorométhoxy.

5. Composé selon l'une des revendications 1 à 4 où R³ est 2-(3,5-bis-trifluorométhylphényl)éthyle.

6. Composé selon l'une des revendications 1 à 5 où le groupe -NR³R⁴ est

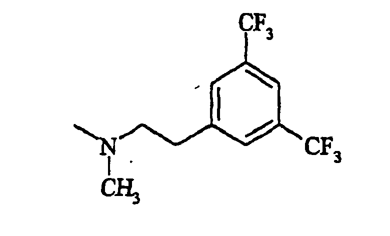

7. Composé selon l'une des revendications 1 à 6 où
R¹ représente un groupe C₁-C₃-alkyle, phényle ou C₁-C₃-alcoxyphényle,
X représente NH, et
R² représente un atome d'hydrogène.

8. Composé selon l'une des revendications 1 à 6 où
R¹ et R² combinés représentent un groupe éthylène-1,2-diyle, 1-oxoéthylène-1,2-diyle, propylène-1,3-diyle, oxopropylène-1,3-diyle ou 1-oxobutylène-1,3-diyle, et
X représente O, NH ou NCH₃.

9. Composé selon l'une des revendications 1 à 8 choisi parmi les composés des formules

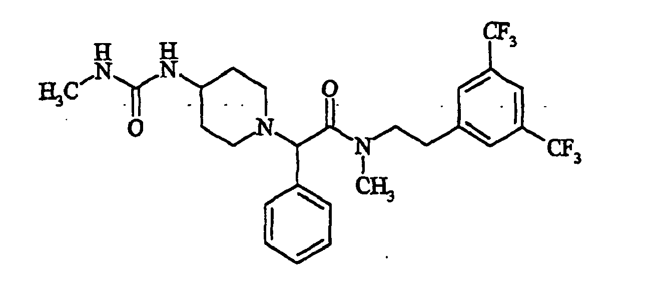

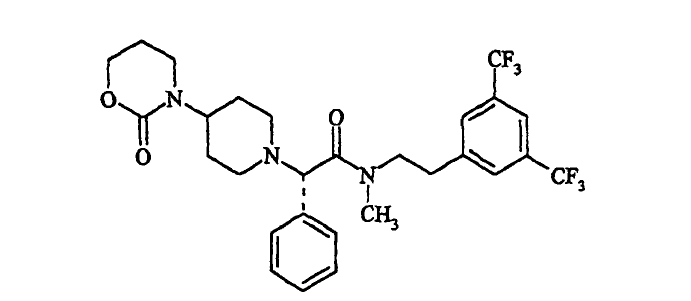

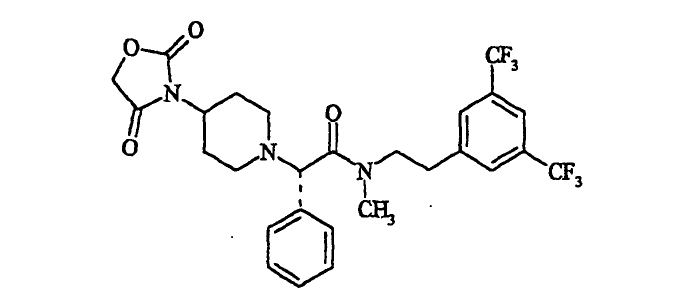

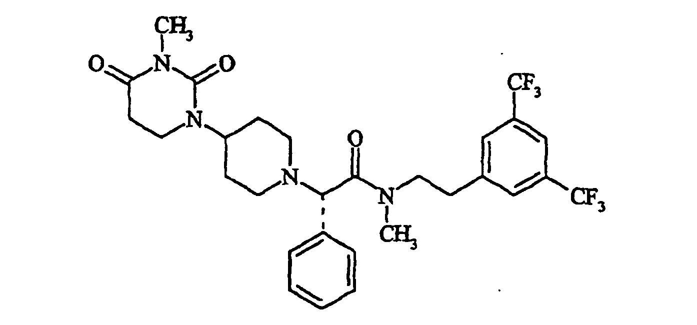

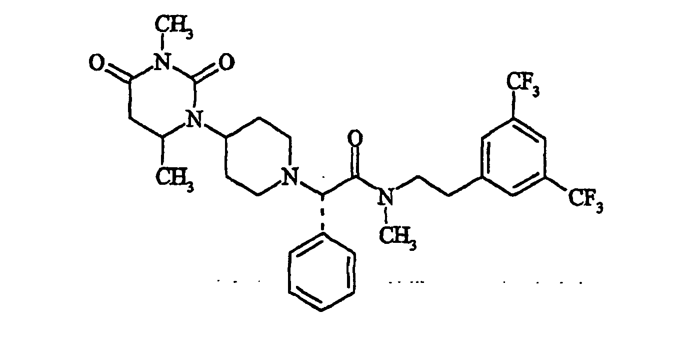

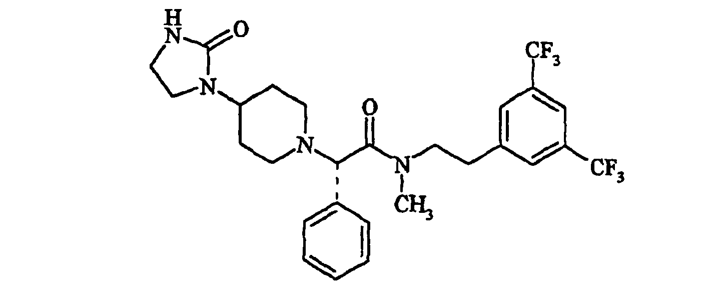

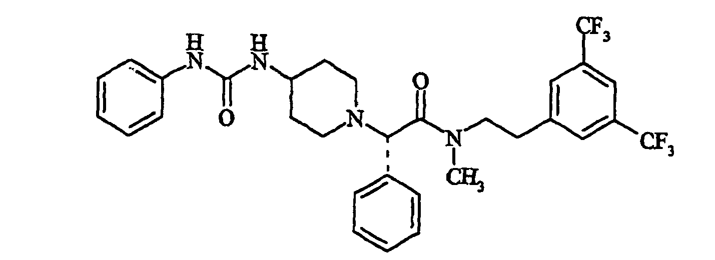

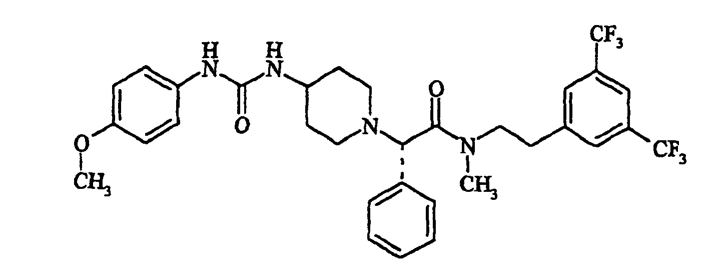

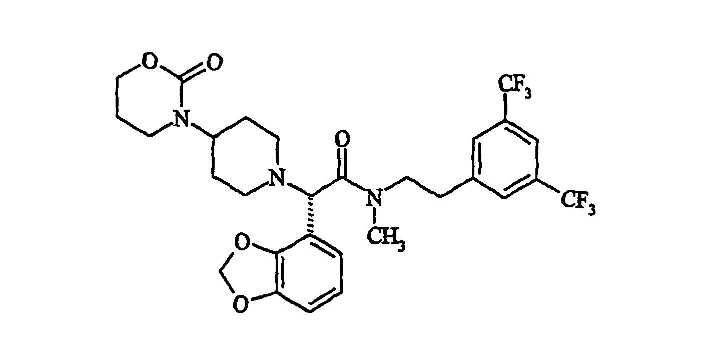

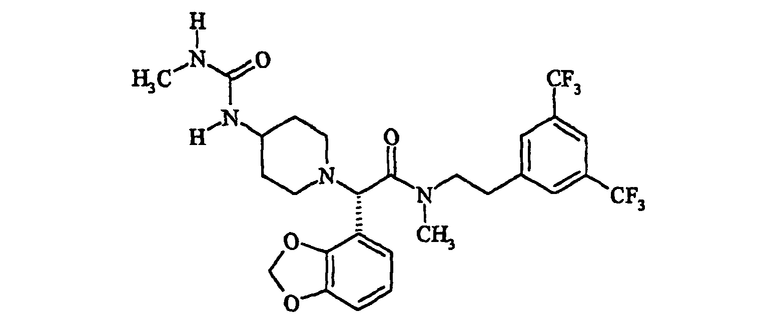

10. Composé selon l'une des revendications 1 à 9 destiné à être utilisé comme médicament.

11. Procédé pour préparer un composé de formule I selon l'une des revendications 1 à 9 **caractérisé en ce que** l'on fait réagir un amide de formule II 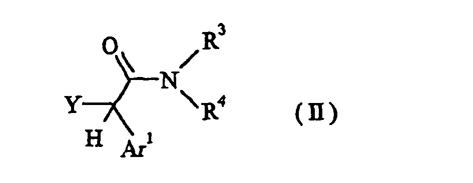
où Ar¹, R³ et R⁴ présentent les significations indiquées dans les revendications 1 à 9, et Y représente un groupe partant approprié,
dans un solvant inerte éventuellement en présence d'une base avec une pipéridine de formule générale III 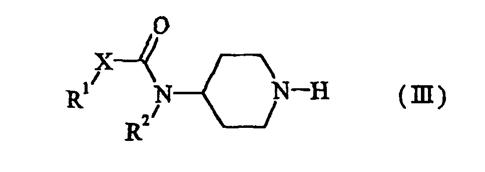
où R¹, R² et X présentent les significations indiquées dans les revendications 1 à 9.

12. Préparation pharmaceutique contenant un composé selon l'une des revendications 1 à 9 et des supports et excipients pharmaceutiquement acceptables.

13. Utilisation d'un composé selon l'une des revendications 1 à 9 pour la production d'une préparation pharmaceutique pour la thérapie et la prévention des maladies à médiation par des neurokinines.
